# EUROPEAN PATENT SPECIFICATION

(11) **EP 4 061 335 B1**
(45) Date of publication and mention of the grant of the patent: **19.11.2025**
(21) Application number: 20890742.8
(22) Date of filing: 19.11.2020
(51) Int. Cl.: A61K 9/00, A61K 9/12, A61M 11/00

(54) **HIGH-THROUGHPUT MONODISPERSE AEROSOLS OF MICRO-SHELL PARTICLES**
MONODISPERSE AEROSOLE AUS MIKROSCHALENPARTIKELN MIT HOHEM DURCHSATZ
AÉROSOLS MONODISPERSÉS À HAUT DÉBIT DE PARTICULES MICROENCAPSULÉES

(30) Priority: 22.11.2019 US 201962939179 P
(43) Date of publication of application: 28.09.2022
(73) Proprietor: The Trustees of Princeton University, Princeton, NJ 08544 (US)
(72) Inventor: MEZHERICHER, Maksym, Princeton, NJ 08540 (US); STONE, Howard, A., Princeton, NJ 08540 (US)
(74) Representative: FRKelly
(86) International application number: PCT/US2020/061186
(87) International publication number: WO 2021/102085

(56) References cited:
- WO-A1-96/09846
- WO-A2-2013/001309
- US-A- 5 645 124
- US-A1- 2002 003 312
- US-A1- 2008 072 895
- US-A1- 2009 098 168
- US-B1- 6 228 346

## Description

### CROSS-REFERENCE TO RELATED APPLICATIONS

This application claims priority to U.S. Provisional Patent Application No. 62/939,179, filed November 22, 2019, which is hereby incorporated in its entirety by reference.

### TECHNICAL FIELD

The present invention relates generally to the generation of aerosol particles, and specifically to methods and systems for high throughput generation of aerosols with stable, monodispersed, submillimeter-sized micro-shell particles using gas jets and falling films.

### BACKGROUND

Aerosols are useful in many industries, including medical, pharmaceutical, and food industries. In the medical field, aerosols can be useful for the delivery of therapeutic or diagnostic agents, as they avoid problems associated with other delivery methods such as oral administration (acidic environment of body/stomach, the ability of the agent to pass through the walls of the intestines, the liver's metabolizing of the agents, etc.) or injection (pain, risk of infection, hazardous waste generation, etc.). Aerosols can deliver an agent directly to a target area, such as the eye, or the nasal or respiratory tract. The latter can be an efficient way of direct delivering drugs to treat COVID-19 patients.

There are multiple types of aerosols, including, e.g., liquid droplets, as well as what are called bubble aerosols. Bubble aerosols consist of liquid particles having a thin liquid shell surrounding a gas core, which are suspended in a carrier gas flow or environment.

In order for droplet aerosols to be useful in biomedical applications, however, either the aerosols must be generated at the point of use, or they must have long-term stability. The two types of currently functional aerosols include aerosols of liquid droplets, generated by nebulizers and metered-dose inhalers, and aerosols of dry particles, which are separately produced and loaded into dry powder inhalers. Both nebulizers and metered-dose inhalers are point of use devices that create aerosols of liquid droplets by forcing a liquid suspension through an orifice to generates an aerosol. Neither has long-term stability - both generate aerosols that need to last seconds, at most. Additional disadvantages of those devices include generation of polydisperse droplet size distributions, low particle deposition in the lower airways of the human respiratory system, and the difference in the concentration of a therapeutic agent between delivered droplets compared to an initial solution, which all pose risks of insufficient or excessive medicine delivery. And neither of those two devices can generate aerosols of uniform-diameter particles, as well as liquid micro-shells (aerosol bubbles), which can be used as drug carriers and instantly solidified by either solvent evaporation, freezing or a chemical reaction. In addition, none of the current devices generating aerosols of droplets has additional functionality of formation of fine monodisperse liquid or solid foams simply by collecting liquid micro-shells from the generated aerosol on a surface or in a vessel, while the liquid walls of particles of adhere to each other and then can solidify due to solvent evaporation, freezing or polymerization.

Therefore, a system and method for generating stable aerosols of uniform-diameter liquid micro-shells are useful and desirable.

### BRIEF SUMMARY

A first aspect of the present disclosure is a system for generating monodisperse bubble aerosols, consisting of uniform-diameter submillimeter-diameter particles having a thin liquid shell surrounding a gas core. The system includes a frame configured to create a falling film having a thickness less than 20 µm, where the falling film has a first end (a top), a second end (a bottom), and an intermediate portion. The system also includes a small nozzle configured to direct a gas towards the intermediate portion of the falling film, preferably in a direction perpendicular to a surface of the falling film, and a controller that is adapted to control the pressure of the gas provided to the nozzle, the pressure being between 10 and 1500 mbar.

The system may contain a pump configured to provide a liquid to the top portion of the falling film at a controlled rate, a camera directed at the intermediate portion, and/or a container to collect the liquid from the bottom of the falling film. The liquid used to form the falling film may optionally have a viscosity less than 100 mPa·s at 25 °C, and may optionally contain an additional material that is suspended or dissolved in the liquid, such as an active pharmaceutical ingredient (API). The supplied gas directed into the nozzle may optionally contain suspended micron- or nano-sized particles or droplets, which may comprise an API. The nozzle may optionally have an inner diameter of less than 1 mm, or less than 1 µm. Optionally, the system may utilize a bank of nozzles blowing in parallel on one or more falling films. Optionally, a carrier gas is supplied to dilute or direct the flow of the generated bubble aerosol. The system preferably creates monodisperse aerosol bubbles, which are uniform-diameter particles having a thin liquid shell surrounding a gas core. Thus, aerosols of bubbles are essentially aerosols of liquid micro-shells, and those two terms are used interchangeably through this text. Optionally, the generated liquid shells may have an average outer diameter (or a maximum outer diameter) less than 1 mm. Optionally, the produced liquid shells may have a wall thickness of less than 5 µm. Optionally, the film is created by either pumping a liquid containing small amount of surfactant to the top of a vertically-oriented frame and allowing the liquid to create the film as it falls, or periodically dipping the frame into a liquid containing surfactant. Optionally, the system includes a second falling film, and optionally the aerosol bubbles that are formed from the first falling film then pass through the second falling film. This process optionally creates a multi-layer shell structure, around the central core, which initially comprises the gas.

A second aspect of the present disclosure is drawn to a method for generating bubble aerosols, which preferably relies on the system described previously. The method involves creating a falling film from a liquid containing a dissolved surfactant, the falling film having a thickness of less than 20 µm; and then generating a plurality of liquid micro-shell particles by providing a gas at a pressure of between 10 and 1500 mbar to a nozzle configured to direct the gas through the falling film. The method may optionally include solidification of aerosol bubbles, by either solvent evaporation, freezing or chemical reaction like polymerization, collecting the plurality of aerosol bubbles, and/or generating a solid foam material from the plurality of aerosol bubbles. Optionally, the method involves generating between 1500 and 5000 uniform-diameter liquid micro-shells are generated per second per nozzle.

### BRIEF DESCRIPTION OF DRAWINGS

Figure 1 is a depiction of an embodiment of the disclosed system.
Figure 2 is a cross-section of an alternate embodiment of a portion of the disclosed system, focusing on the nozzles, falling film, and formed aerosol bubbles.
Figure 3A is a cross-section of an alternate embodiment of a portion of the disclosed system, focusing on a nozzle, falling films, and formed aerosol bubbles.
Figure 3B is a cross-section of a core-shell construction of formed aerosol bubbles.
Figure 4 is a graph, illustrating the relationship between the nozzle pressures, flow rates for forming the film, and the operating regimes of the system.
Figure 5 is a graph, illustrating the relationship between the feed flow rate and the film thickness, and specifically the relationship for three different water and dish soap mixtures.
Figure 6 is a depiction of the process of liquid micro-shells production, including a nozzle (black horizontal line in upper left, about 1 mm long in practice) directing air towards a falling film (dark vertical line about 2 cm from the left edge in the image, about 1 mm away from the end of the nozzle in practice) forming a steady stream of monodisperse aerosol bubbles (dark spheres, generally forming within 2 cm of the falling film, about 2 mm in practice).
Figure 7 is a schematic diagram illustrating various embodiments for the production of high-throughput monodisperse aerosols of micro-shell particles and fine foams for health applications.

### DETAILED DESCRIPTION

The present invention is drawn to a system and method for creating stable, monodisperse aerosol of micro-shell particles, the aerosols comprising particles having liquid shell of micron-scale thickness surrounding a gas core (sometimes referred to herein as "aerosol bubbles").

As used herein, "monodisperse" refers to particles having substantially uniform outer dimensions. In some embodiments, the polydispersity index (PI), a measure of the heterogeneity of a sample based on size, is used to reflect the dispersity of the particles. Assuming a gaussian distribution of particle sizes, the PI is generally understood as the square of (standard deviation / mean diameter of the particles). The PI can be obtained from instruments that use dynamic light scattering (DLS) or determined from electron micrographs. International standards organizations (ISOs) have established that PI values < 0.05 (5%) indicate a highly monodisperse sample, while values > 0.7 (70%) indicate a broad distribution (e.g., polydisperse) of particle sizes (ISO standards ISO 22,412:2017 and ISO 22,412:2017). PI values < 0.1 (10%) are generally understood as being monodisperse. As such, in preferred embodiments, the monodisperse particles have PI values < 0.05, < 0.04, <0.03, <0.02, or <0.01.

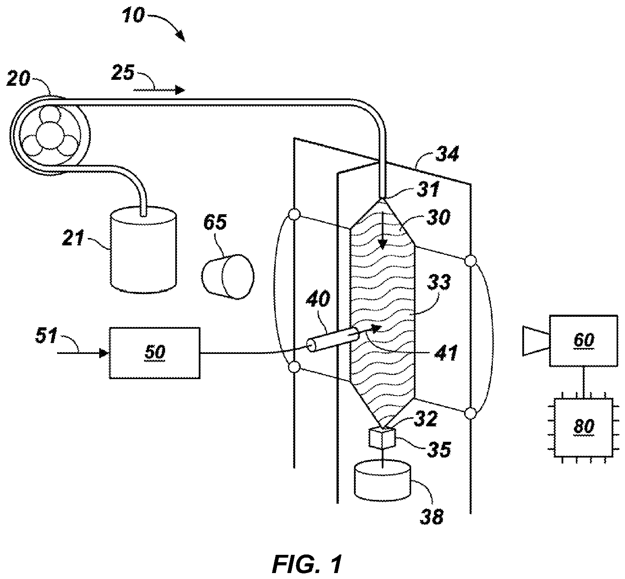

The system can best be understood referring to Figs. 1-4. As can be seen in Fig. 1, the system **10** can generally be described as requiring a nozzle **40** to be positioned so as to direct a stream **41** of a gas toward and through a falling film 30, in order to form aerosol bubbles.

### Nozzle

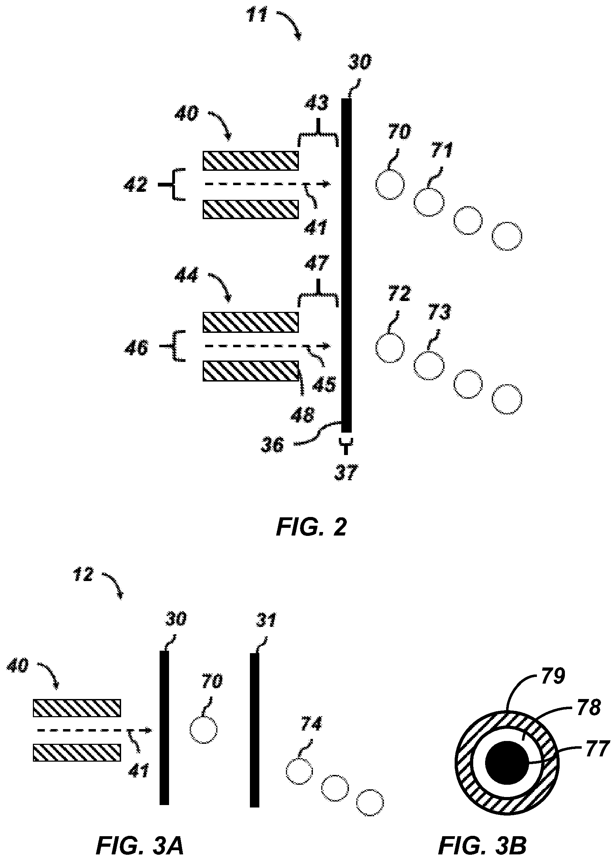

The nozzle **40** is shown in Fig. 1 as being a single nozzle. However, the system may contain multiple nozzles, each of which can be configured and controlled independently. In some embodiments, a plurality of nozzles, which may be a bank of nozzles (nozzles that are connected and/or otherwise set at a fixed distance from each other), can be directed towards a falling film **30.** For example, referring to Fig. 2, the system **11** may contain two nozzles **40, 44** spaced apart, the end of the first nozzle **40** has a first inner diameter **42** and the end of the second nozzle **44** has a second inner diameter **46.** Note that the inner diameter should be measured on the end **48** closest to a first surface **36** of the falling film **30.** The inner diameters of each nozzle may be the same, or may be different, depending on the aerosol bubble output desired. The ends of each nozzle are spaced a distance **43, 47** from the falling film **30,** and are oriented to direct a stream of gas **41, 45** towards and through the falling film **30.** The first nozzle **40** forms a first stream of monodisperse aerosol bubbles **70, 71.** The second nozzle **44** forms a second stream of monodisperse aerosol bubbles **72, 73.** These streams of aerosol bubbles may have identical or different mean particle sizes. In preferred embodiments, each stream of aerosol bubbles has a mean particle size deviating within 10% difference of all other streams of aerosol bubbles.

Referring to Fig. 2, the nozzles **40, 44** in any system can theoretically have almost any inner diameter **42, 46.** Without adjusting any other characteristic of the system, the mean diameter of the formed aerosol bubbles will generally vary linearly with the inner diameter of the nozzle. To form submillimeter aerosol bubbles, the inner diameter of the nozzle should preferably be less than 1 mm. To form aerosol bubbles with diameter near or below one micrometer, the inner diameter of the nozzle should preferably be less than 1 µm. To form nanoparticles, the inner diameter of the nozzle should preferably be less than 100 nm.

Referring to Fig. 2, typically, as the inner diameter **42, 46** is reduced, the smaller the space or distance **43, 47** there should be between the end of the nozzle **40, 44** and the falling film **30.** In one example, glass nozzles with an inner diameter of between 0.1 and 0.5 µm (100 to 500 nm), were placed with the ends of the nozzle within 1 - 5 µm from the falling film, while nozzles with an inner diameter of about 0.1 mm (100 µm) was placed approximately 0.9 - 1.5 mm from the film. In some embodiments, the space between the ends of the nozzle and the film is between 5 and 20 times the inner diameter, although the distances will vary based on pressure and film characteristics (e.g., film thickness for a given liquid composition).

The nozzles utilized in the disclosed systems preferably have a circular cross-section, and the end of each nozzle is preferably a flat surface perpendicular to the direction the gas is flowing through the nozzle. Preferably the nozzles do not contain a bevel, such as those seen on regular hypodermic needles.

The nozzles may be of almost any material. For example, in some embodiments, the walls defining a nozzle are comprised of a metal (such as stainless steel, platinum, etc.) or a glass (such as a silicon dioxide glass).

In some embodiments, the nozzles **40** may be attached to a frame **34,** positioned on one side of the falling film **30.** In other embodiments, the nozzle is not attached to the frame **34.** In some embodiments, the nozzles are in a fixed position and fixed orientation, while in others, the nozzles may vary in position, orientation, or both. For example, in some embodiments, a nozzle can be connected to spatial positioners driven by motors which control the location of a given nozzle's 2-D location (in a plane parallel to the film), and/or spatial positioners driven by motors which control the orientation of a given nozzle with respect to the film. In preferred embodiments, each nozzle **40, 44** is oriented such that the stream **41, 45** is oriented 90° (normal/perpendicular) to a first surface **36** of the falling film **30.** In other embodiments, one or more streams **41, 45** deviates from normal to the first surface **36** by ≤ 5°, ≤ 10°, ≤ 15°, ≤ 20°, ≤ 25°, ≤ 30°, ≤ 35°, ≤ 40°, or ≤ 45° in any direction. Said differently, while the nozzles preferably direct a gas perpendicular to the surface of the film, it may sometimes be advantageous for the nozzles to direct the gas at a slight or moderate angle relative to the surface of the film.

### Gas

The nozzle **40** is preferably connected to a flow control unit **50,** such as a microfluidic flow control unit, that controls the flow of a gas from a source **51** to the nozzle **40.** Flow control units are well known in the art and are commercially available in a variety of configurations. The flow control unit **50** is preferably adapted to control the pressure of the gas provided to each nozzle **40.**

Skilled artisans will recognize that, to form aerosol bubbles, the pressure of the gas being provided to nozzle **40** is at least partially dependent on the characteristics of the falling film and the liquid used to form the falling film **30.** For example, a thin film will require less pressure than a thicker film, and a liquid's viscosity and surface tension will also impact how much pressure is required. In some embodiments, the pressure existing the flow control unit **50** is ≥ 10 mbar, ≥ 20 mbar, ≥ 30 mbar, ≥ 40 mbar, ≥ 50 mbar, or ≥ 100 mbar, and is ≤ 1500 mbar, ≤ 1250 mbar, ≤ 1000 mbar, ≤ 800 mbar, or ≤ 600 mbar, and all combinations thereof. In some embodiments, the pressure p is 10 mbar ≤ p ≤ 1500 mbar, while in other embodiments, 50 mbar ≤ p ≤ 1000 mbar, and in still other embodiments, 100 ≤ p ≤ 800 mbar.

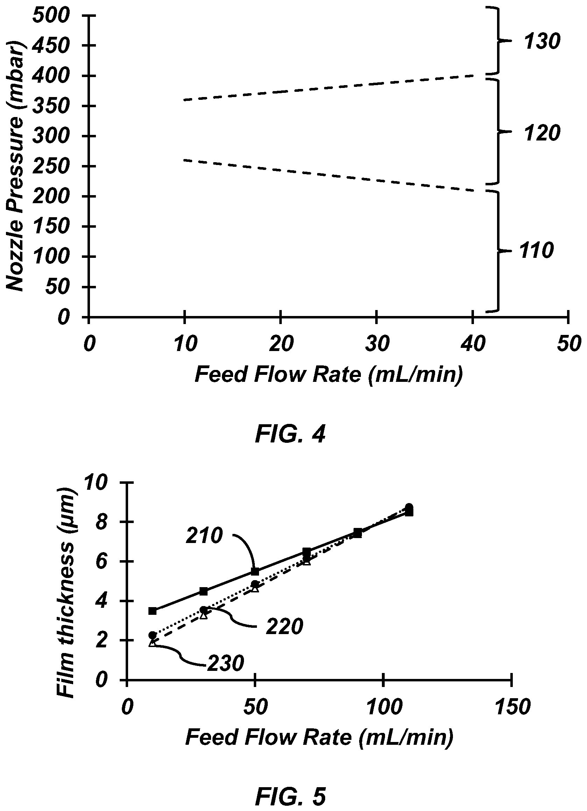

Referring briefly to Fig. 4, a graph showing an example of how the nozzle pressure and the flow rate for the liquid forming the falling film (*i.e.*, the feed flow rate) impact the formation of bubble aerosols. In this specific example, the gas was compressed air, the nozzle had a diameter of 110 µm, and the liquid was 1.5% by volume of dish soap in water. As can be seen, when the pressure is low, the formation process falls into a first regime **110.** In the first regime **110,** a cavity is formed in the liquid film, but no aerosol bubbles are formed. As the pressure increases, eventually the system reaches a second regime **120,** and high-throughput formation of uniform-diameter aerosol bubbles (aerosol particles having liquid micro-shell surrounding a gas core) occurs. This is the regime this present invention is intended to utilize. If pressure is increased still further, the system can reach a third regime **130,** where liquid film bursting due to overpressure occurs. In this regime, aerosol bubbles do not form, and instead, the liquid film is ruptured locally.

The gas that is used to create the aerosol bubble can include any gas or gas mixture and may also include a liquid or solid micro/nano-size particles suspended in the supplied gas or gas mixture. Typically, the gas or gas mixture will comprise a carrier gas that does not substantially react with other components in the system. This gas or gas mixture may comprise, e.g., air, CO₂, N₂, or O₂, or noble gases like He, Ar, and the gas or gas mixture may or may not have been passed through filters prior to being used by the system.

In some embodiments, if the gas contains suspended micron- or nano-sized particles or droplets, the suspended particles or droplets comprise an active pharmaceutical ingredient (API), or biopharmaceutical materials, such as proteins or engineered microorganisms, etc. Other suspended materials or droplets may comprise a catalyst, food, or cosmetic ingredient, etc. For example, droplets can be used to encapsulate an active sunscreen ingredient, such as octocrylene, avobenzone, etc., and that encapsulated material can then be used in a sunscreen formulation.

In some embodiments, the suspended micron- or nano-sized particles or droplets are introduced to the gas or gas mixture prior to the flow control unit **50.** In some embodiments, the suspended particles or droplets are introduced into the gas stream after the flow control unit **50.** In some cases, this particles or droplets are metered into the gas through a Y-type connection.

In some embodiments, a carrier gas is supplied to dilute or direct the flow of the generated aerosol of micro-shell particles. Such carrier gas can be supplied either as a bulk environmental flow to which all the system shown in Fig. 1 is exposed, or a local flow supplied after thin film **30** to dilute or guide the flow of generated micro-shell particles. In some embodiments, the carrier gas supplied after thin film **30** can be hot dry air to promote rapid solvent evaporation from the generated liquid micro-shells and their conversion into solid micro-shells. In some embodiments, the carrier gas can be moist air with 100% relative humidity supplied to prevent evaporation of solvent from the generated liquid micro-shells and promote their transportation to long distances. In some embodiments, the carrier gas can be saturated vapor of nitrogen or saturated vapor of carbon dioxide, which is supplied to solidify the generated liquid micro-shells by freezing them. In some embodiments, the carrier gas can be a monomer vapor, which is combined with a catalyst or an oxidant included in the micro-shells to promote chemical reaction of gas phase polymerization. In some embodiments, the carrier gas can be a catalyst gas, which is combined with a monomer included in the micro-shells to promote chemical reaction of polymerization.

In some embodiments, the gas may comprise non-pharmaceutical and non-medicinal agents, such as scent agents. In some embodiments, the gas comprises such scent agents in order to be used to encapsulate a desirable or masking scent (such as a fragrance). That is, in some embodiments, the process results in a plurality of aerosol bubbles encapsulating a fragrance, where the fragrance would generally not be released until the outer shell was dissolved, popped, etc. In some embodiments, the desirable scent is not an aesthetically pleasing scent; that is, in some embodiments, the scent agent is a malodorant.

### Falling Film

The falling film **30** is typically formed on a frame **34,** that uses, e.g., wires, to hold and provide an area for the film to form. A liquid **21** (including, *e.g*., as a pure liquid, a solution, an emulsion, or a liquid containing suspended particles) passes to a pump **20,** which controls the rate of the liquid **21** flowing to the falling film **30.** The falling film **30** generally has a top portion **31,** a bottom portion **32,** and an intermediate portion **33.** The liquid **21** is pumped to the top portion **31.** Gravity causes the film to fall from the top portion **31** towards the bottom portion **32,** typically conforming to a shape defined by wires connected to the frame **34.** In some embodiments, a tensioning weight **35** is attached to the wires near the bottom portion **32,** to aid in maintaining a uniform falling film. The liquid **21,** after passing through the falling film **30** from the top portion **31** to the bottom portion **32,** then typically drains into a collection device **38,** which is typically an appropriately-sized container (*e.g*., a beaker, a drum, etc.) or funnel. In some cases, the liquid **21** from the collection device **38** is recycled, pumped back to the top portion **31** of the falling film **30.** In some cases, the recycling is done in-line, and the collected liquid is then pumped back to the top portion **31** of the falling film **30.**

The falling film as shown in Fig. 1 has a roughly hexagonal shape - the top portion **31** expands from a near-point at the top of falling film **30** to a predefined width. It is in this top portion that the film converts from a stream in, e.g., a connecting tube or pipe, becoming the desired thin film. The intermediate portion **33** then has a length at which it falls at that predefined width. It is in this intermediate portion that the film is the most uniform. The bottom portion **32** is the inverse of the top portion **31,** reducing from the predefined width to a near-point at the bottom of the falling film **30.**

In some embodiments, the film is initially generated using a relatively slow flow rate, and after the entire film is generated, the flow rate is increased until the desired film thickness is achieved.

Generally, the flow rate of the liquid can be used to control the thickness of the falling film. As seen in, *e.g.*, Fig. 5, for three different combinations of water and dish soap, the thickness of the films increases in a substantially linear manner as the flow rate increased from about 10 mL/min to about 110 mL/min. The three different compositions included 0.5% v/v dish soap **210,** 1.5% v/v dish soap **220,** and 3.0% v/v dish soap **230** dissolved in water. The 0.5% v/v dish soap **210** forms a slightly thicker film at lower flow rates, but was slightly less thick than the other compositions at 110 mL/min (about 8.5 µm vs about 8.75 µm). The 1.5% v/v dish soap **220,** and 3.0% v/v dish soap **230** compositions both performed in similar manners, with the 1.5% v/v dish soap **220** forming a very slightly thicker film at lower flow rates.

While the minimum and maximum film thickness of a falling liquid that still allows for aerosol bubble formation will depend on the liquid's composition, films that can form aerosol bubbles will typically have a thickness of less than about 20 µm. In some embodiments, the thickness of the film is ≤ 20 µm, ≤ 18 µm, ≤ 16 µm, ≤ 14 µm, ≤ 12 µm, or ≤ 10 µm, and is ≥ 1 µm, ≥ 3 µm, ≥ 5 µm, or ≥ 10 µm, and all combinations thereof.

The wall thickness of the aerosol bubbles that are formed is at least partially based on the thickness of the falling film. As the film thickness increases, the wall thickness will also generally increase.

Additionally, as will be understood by those of skill in the art, the wall thickness is bound by the diameter of the aerosol bubble. It is physically impossible for a bubble with a 1 µm outer diameter to have 1 µm thick walls, for example. In some embodiments, the thickness of the walls is less than 20%, less than 10%, or less than 5% of the outer diameter of the bubble.

The composition of the liquid can vary, depending on the desired purpose of the aerosol bubble coating to be formed. The liquid will be a liquid. In some embodiments, the liquid comprises water and other water-soluble ingredients, such as alcohols (*e.g*., ethanol, etc.) or polyols (*e.g*., glycerol, etc.). In some embodiments, the liquid is non-aqueous. In some embodiments, the liquid is a solution. In some embodiments, the liquid is an emulsion. In some embodiments, the liquid contains an additional material that is suspended or dissolved in the liquid, such as an active pharmaceutical ingredient (API) or catalyst or monomer or oxidant. In some embodiments, the liquid also comprises a surfactant. The surfactants may be, e.g., pharmaceutically acceptable surfactants, including, e.g., non-ionic surfactants. In some embodiments, the surfactant has an HLB (hydrophilic lipophilic balance) value of from about 4 to about 10, preferably from about 7 to about 9. Surfactants having an HLB value of from about 4 to about 10 suitable for use in the present invention include for example, but are not limited thereto: polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether, polyoxyethylene (3) octylphenyl ether; polyethylene glycol fatty acid esters, e.g. PEG-200 monolaurate, PEG-200 dilaurate, PEG-300 dilaurate, PEG-400 dilaurate, PEG-300 distearate, PEG-300 dioleate; alkylene glycol fatty acid mono esters, e.g. propylene glycol monolaurate (Lauroglycol^{®}); sucrose fatty acid esters, e.g. sucrose monostearate, sucrose distearate, sucrose monolaurate, sucrose dilaurate; or sorbitan fatty acid mono esters such as sorbitan mono laurate (Span^{®} 20), sorbitan monooleate, sorbitan monopalmitate (Span^{®} 40), or sorbitan stearate, or mixtures of one or more thereof.

In some embodiments, the liquid includes other components, such as solvents, solubilizing agents, waxes, colorants, plasticizers, hydrophobic polymers, or hydrophilic polymers. A simple example is a liquid comprising water, 0.1-10% of a surfactant, and 0.1-5% of a hydrophilic polymer (such as polyethylenimine (PEI)).

The nozzles in the system are preferably directed towards the intermediate portion **33** of the falling film **30,** avoiding any thickness or velocity gradients near the edges that might negatively impact uniformity. The length and width dimensions of the intermediate portion **33** can vary significantly. In some cases, the length of intermediate portion **33** is ≤ 200 cm, ≤ 150 cm, ≤ 100 cm, ≤ 50 cm, ≤ 25 cm, ≤ 10 cm, or ≤ 5 cm. In some cases, the width of intermediate portion 33 is less than < 20 cm, ≤ 10 cm, ≤ 8 cm, or ≤ 5 cm, or ≤ 1 cm, or ≤ 0.5 cm In some embodiments, the overall length of the falling film **30** is ≤ 250 cm, ≤ 200 cm, ≤ 150 cm, ≤ 100 cm, ≤ 50 cm, ≤ 25 cm, or ≤ 10 cm, or ≤ 5 cm, or ≤ 1 cm.

In some embodiments, the nozzles are directed towards the center of the intermediate portion **33** of the falling film **30,** ± 25% of the width and/or length. That is, if the film is 8 cm wide and 20 cm long, the nozzles are generally directed at a portion of the film that is 4 cm from the left side of the intermediate portion **33,** ± 2 cm, and 10 cm from the top of the intermediate portion **33,** ± 5 cm. In some embodiments, all nozzles are within this centralized target range. In some embodiments, only some of the nozzles are within this centralized target range.

The system in Figs. 1 and 2 show an aerosol bubble being formed using a single falling film **30.** However, as seen in Fig. 3A, in some embodiments, a system **12** can include multiple falling films **30, 31.** In Fig. 3A, the nozzle **40** directs a stream **41** of a gas towards a first falling film **30.** The aerosol bubble **70** that forms from the air passing through the first film is driven away from the first film **30** towards a second falling film **31.** The momentum of the aerosol bubble **70** drives the aerosol bubble through the second falling film **31,** resulting in a stream of aerosol bubbles **74.** The stream of aerosol bubbles **74** may then pass through additional falling films (not shown). The number of falling films the aerosol bubbles **74** can pass through are limited only by the ability of the aerosol bubble to pass through a given film, which is driven, in part, by the velocity of the aerosol bubbles, thickness of the film and the viscosity and/or surface tensions of the film.

In preferred embodiments, the liquid of each falling film will have a viscosity ≤ 500 mPa·s, ≤ 250 mPa·s, ≤ 200 mPa·s, ≤ 150 mPa·s, or ≤ 100 mPa·s, at 25 °C.

In preferred embodiments, the liquid of each falling film will have a surface tension ≤ 100 mN/m, ≤ 75 mN/m, ≤ 50 mN/m, ≤ 40 mN/m, or ≤ 30 mN/m.

Because each falling film **30, 31** may have different compositions, this allows a skilled artisan to create a layered aerosol bubble structure, or a core-shell particle. Referring briefly to Fig. 3B, each aerosol bubble or core-shell particle can, for example, contain a core **77** that initially comprises the stream **41** of gas being directed at the first falling film **30.** The first shell **78** of the particle initially comprises the first falling film **30.** If no other falling films are utilized, that is the structure of the aerosol bubbles. However, if other falling films are utilized in the system, the second shell **79** of the particle would initially comprise the second falling film **31.** Additional falling films (not shown) would add additional layers as desired. Skilled artisans will recognize that the volatile materials will eventually leave the aerosol bubble, so the starting compositions will not match the final composition of the aerosol bubble.

The use of multiple films also allows the creation of unique particle configurations. In some embodiments, one layer forms a hydrophobic shell, while another layer forms a hydrophilic shell, which can remain "wet" or can be dried. In some embodiments, a first layer uses a polymer material that can be quickly solidified (e.g., by drying , photopolymerization, etc.) to form a solid shell, and then the second falling film the second layer can remain "wet" or can be dried as well. This process can be repeated as long as the aerosol bubble/shell still have enough inertia to pass through a film. So the aerosol bubbles that are created can be a hollow dry multi-layer particle, and inside the central void of such particle there could be a gas and liquid/dry nanoparticles of a useful material, like medical drug, catalyst, food, or cosmetic ingredient, etc.

In preferred embodiments, all aerosol bubbles (shells with thin liquid walls and gas core) that are generated are monodisperse with respect to all other aerosol bubbles generated by all nozzles. In some embodiments, all aerosol bubbles that are generated from each nozzle is monodisperse with respect to the aerosol bubbles generated from that nozzle.

In some embodiments, the diameter (D) of the aerosol bubbles scales with the inner diameter (ID) of the nozzle, such as D = x ID, where generally 1 < x < 10. In some embodiments, 2 < x < 4. For example, with an approximately 110 µm inner diameter nozzle, aerosol bubbles were created that had bubble diameters of around 300 µm. In other examples using the same nozzle, the bubble diameters could be varied from between 300 and 800 µm, depending on various flow and liquid conditions.

In some embodiments, the generated aerosol bubbles have an average diameter (D_{avg}) < 1 mm, < 750 µm, < 500 µm, < 250 µm, or < 100 µm. In some embodiments, the generated aerosol bubbles have a maximum diameter (Dₘₐₓ) < 1 mm, < 750 µm, < 500 µm, < 250 µm, or < 100 µm, or < 50 µm, or < 20 µm, or < 10 µm, or < 5 µm, or < 1 µm.

In preferred embodiments, the thickness of each shell or wall of the produced aerosol bubbles is ≤ 5 µm, ≤ 4 µm, or ≤ 3 µm, or < 2.5 µm, and ≥ 0.5 µm, ≥ 1 µm, ≥ 1.25 µm, or ≥ 1.5 µm, and any combination thereof. In preferred embodiments, the average thickness of each shell or wall of the produced aerosol bubbles is 0.5 µm ± 0.12 µm, 1 µm ± 0.25 µm, 2 µm± 0.25 µm, 3 µm ± 0.25 µm. These thin walls can enable rapid evaporation of solvents in the bubble walls.

Because of the small diameters and relatively thin walls, each aerosol bubble particle is very light, generally weighing ≤ 20 µg, ≤ 15 µg, ≤ 10 µg, or ≤ 5 µg, or ≤ 1 µg. This can enable precise delivery of materials, as very small payloads can be directed to a target location.

The throughput of the produced aerosol bubbles can vary dramatically. For example, in some experiments, the particle throughput was 1500-3500 aerosol bubbles per second, which translates to 10-30 mg/s. In some embodiments, the particle throughput was 1500-5000 aerosol bubbles per second per nozzle.

Given that thousands of aerosol bubbles can be generated quickly, these streams of bubbles can be collected (e.g., in a container, on a surface, etc.) and/or manipulated as desired.

In some instances, the aerosol bubbles are further dried and/or solidified, which may occur while the aerosol bubbles are in flight. In one example, a gas blowing on the film contains suspended micron/nano particles in it; these particles are then encapsulated by the aerosol bubble walls, which can solidify or not (depending on the solvent system, etc.) during their flight.

Alternatively, in some examples, polymers in a shell of the particles are cross-linked by exposed the aerosol bubbles to ultraviolet (UV) light. In some examples, the collected aerosol bubbles adhere to each other, and polymerize on the surface of the container or substrate that they were collected on. In some embodiments, the carrier gas can be saturated vapor of nitrogen or saturated vapor of carbon dioxide, which is supplied to solidify the generated liquid micro-shells by freezing them. In some embodiments, the carrier gas can be a monomer vapor, which is combined with a catalyst or an oxidant included in the micro-shells to promote chemical reaction of gas phase polymerization. In some embodiments, the carrier gas can be a catalyst gas, which is combined with a monomer included in the micro-shells to promote chemical reaction of polymerization.

In some embodiments, the process of collecting the aerosol bubbles results in a foam being generated on a surface or in a container (the bubbles will generally adhere to each other to create a foam with monodispersed gas bubbles), and the foam can remain liquid or solidify depending on the solvent in a falling film, and/or the drying and/or solidification process.

Bubbles in aerosol can also be used as transport agents for material delivery. That is, in some embodiments, the aerosol bubbles are configured to pop or dissolve when exposed to certain environments, which then release any materials contained within.

Additionally, while in preferred embodiments, the film is created by pumping the fluid (such as a liquid comprising a surfactant) to the top of a vertically-oriented frame and allowing the liquid to create the film as it falls, alternative approaches can be used. For example, in some cases, the film is created by periodically dipping frame into a liquid with a surfactant.

### Other components

Referring back to Fig. 1, the system may also comprise a camera **60** (which includes any known image sensor) and a light source **65,** and may also contain a processor **80** that is configured to control the camera **60,** the light source **65,** the pump **20,** and/or the flow control unit **50.**

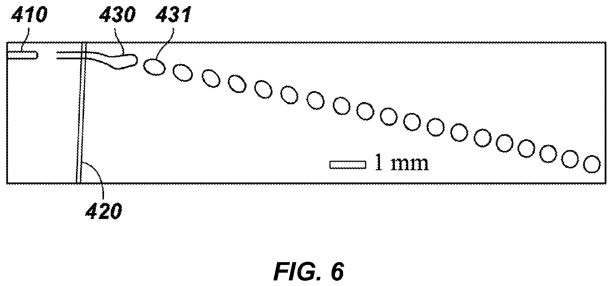

The camera **60,** if used, will preferably be directed to capture side / cross-sectional images of the intermediate portion **33** of the falling film **30,** which during operations can result in images similar to the depiction shown in Fig. 6. There, the nozzle **410** can be seen directing a gas at falling film **420.** A string of formed monodisperse bubbles **431** can be seen, as well as a bubble in the process of being formed **430.**

In some embodiments, the light source **65** can be used to illuminate the falling film and/or aerosol bubbles, allowing the camera to capture better pictures of the film and/or aerosol bubbles.

In some embodiments, the light source **65** can be producing electromagnetic waves of an ultraviolet spectrum and a photopolymer is supplied with the solution **21** to form the thin film **30,** so that when the ultraviolet light **65** is directed onto the generated liquid micro-shells, the light initiates and propagates a photopolymerization reaction for a photopolymer contained in the walls of the generated micro-shells.

In some embodiments, the processor **80** processes images captured by the camera **60** and makes determinations as to the thickness **37** of the falling film **30,** and causes the pump **20** to adjust the rate the liquid is pumped to the falling film **30** to adjust the thickness of the film to reach a desired target or target range.

In some embodiments, the processor **80** processes images captured by the camera **60,** by identifying aerosol bubbles **70, 71, 72, 73** and making at least one determination as to the throughput of the aerosol bubbles. The processor **70** then can cause the flow control unit **50** to adjust the flow rates of the gas one or more of the nozzles **40, 44,** in order to adjust the throughput of the aerosol bubbles to a desired target or target range. If no aerosol bubbles are detected, this may include identifying if the nozzle is operating in the first regime **110** or the third regime **130,** and adjusting the flow rate up or down, respectively, until aerosol bubbles appear.

### Example

In an experimental setup, soap solutions were used made by mixing a dishwashing liquid (surfactant), water and glycerol, that allowed production of falling soap films with thickness 2-15 µm, length 1.3 m and width 8 cm capable of living for tens of minutes. Standard blunt hypodermic needles with internal diameters 90-1000 µm were used as nozzles to provide an air jet from supplied air pressurized within the range 10-1000 mbar. The air jet out of the nozzle was blown perpendicularly to the film. Gradually increasing the air pressure before the nozzle, the three main regimes of gas jet-film interaction were observed, as shown in Fig. 4 (the first regime **110,** second region **120,** and third regime **130).** In the first region **110,** formation of a cavity in the thin film was observed. In the second region **120,** production of aerosol bubbles was observed. In the third region **130,** a regime of thin film bursting was found. In addition, for the second region **120,** bubble production was found to have two sub-regimes: 1) Unstable/polydisperse bubbling, when the air pressure before the nozzle was approximately up to 10-15% higher than the boundary pressure of the region **110;** 2) For the nozzle pressures above that sub-regime and up to the nozzle pressures corresponding to the lower boundary of the region **130,** stable production of uniform-diameter aerosol bubbles was found with a) bubble diameter controlled by a nozzle inner diameter, and b) bubble production rate (throughput) controlled by air pressure supplied to a nozzle of given inner diameter. It was found that an optimal nozzle pressure for the stable production of uniform-diameter aerosol bubbles can be established as approximately half of the sum of the nozzle pressures corresponding to the upper pressure boundary of the region **110** and lower pressure boundary of the region **130.**

Thus, a preferred method for generating aerosols of particles having liquid shell of micron-scale thickness surrounding a gas core (i.e., aerosol bubbles). The process generally involves providing a system as described above, creating a falling film from a liquid containing a dissolved surfactant, the falling film having a thickness of less than 20 µm, then generating a plurality of aerosol bubbles by providing a gas at a pressure of between 10 mbar and 1500 mbar to a nozzle configured to direct the gas through the falling film. A skilled artisan will recognize that some adjustment for the gas flow rate and/or thickness of the film may need to be taken into account in order to stay within the second regime **120** and form a stream of monodisperse aerosol bubbles. In some embodiments, the liquid for the falling film contains the dissolved surfactant and at least one additional material that is suspended or dissolved in the liquid, as described above.

The method may also involve, for example, drying and/or solidifying the aerosol bubbles, generating a solid foam material from the plurality of aerosol bubbles, and collecting the plurality of aerosol bubbles, such as in a container or on a surface.

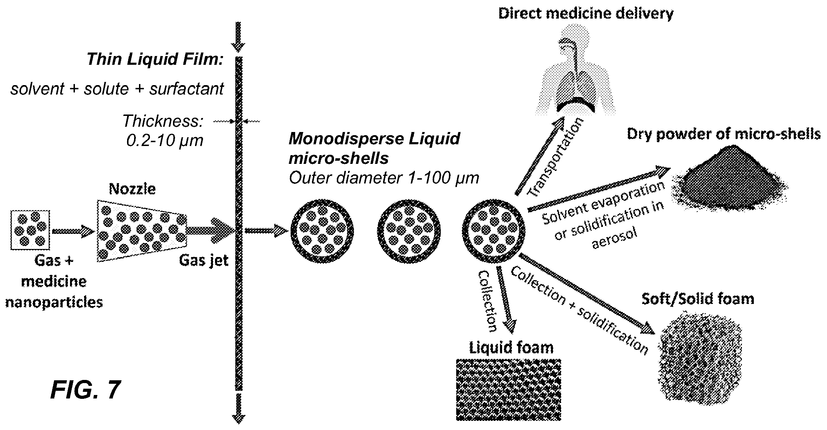

Referring to Fig. 7, embodiments of such method can be visualized. As seen in Fig. 7, a gas containing suspended medicinal nanoparticles is provided to a nozzle, which directs the gas jet towards a thin liquid film. Here, the thin liquid film is shown as being 0.2-10 µm thick, and comprising a solvent, solute, and surfactant. This results in the formation of monodisperse liquid micro-shells (e.g., aerosol bubbles) that encapsulate the medicinal nanoparticles. These aerosol bubbles are shown as having a substantially uniform outer diameter of 1-100 µm. At that point, four different options are illustrated, depending on the desired result.

The first option involves the direct delivery of the medicine. That is, the aerosol bubbles are transported to a patient, who then can have the medicine administered (via, e.g., inhalation). In one embodiment, the aerosol bubbles are directed through tubes to, e.g., a mask, which allows the medicine to be inhaled directly.

The second option is to form a dry powder of micro-shells. This option will generally involve evaporation of the solvent and/or solidification of the liquid micro-shell in the aerosol via known techniques.

The third option is to create a soft or solid foam, which involves the collection of the liquid micro-shells and optionally directing the collected liquid micro-shells to form a particular shape, and then solidifying them.

The fourth option is to generate a liquid foam material, which involves collecting the liquid micro-shells, and optionally directing the collected liquid micro-shells to form a particular shape.

In some embodiments, the falling film is created by pumping a liquid, at a controlled rate, to the top portion of the falling film. In some embodiments, images of the aerosol bubbles being produced are captured by a camera. In some embodiments, a container or funnel is used to capture liquid leaving the bottom of the falling film. In some embodiments, the liquid forming the falling film has a viscosity less than 100 mPa·s at 25 °C. In some embodiments, the liquid forming the falling film contains an additional material that is suspended or dissolved in the liquid, such as an active pharmaceutical ingredient (API). In some embodiments, the gas being provided contains suspended micron- or nano-sized particles or droplets, such as an API. In some embodiments, the nozzle has an inner diameter < 1 mm, or < 1 µm. In some embodiments, a bank of nozzles is used to blow the gas, in parallel, on the falling film. In some embodiments, a carrier gas is supplied to dilute or direct the flow of the generated bubble aerosol. In some embodiments, the aerosol bubbles are monodisperse bubbles, and/or have an average diameter less than 1 mm and/or a maximum diameter less than 1 mm, and/or a wall thickness of less than 5 µm. In some embodiments, the film is created by either pumping liquid comprising a surfactant to the top of a vertically-oriented frame and allowing the liquid to create the film as it falls, or periodically dipping frame into liquid with surfactant.

The disclosed systems and methods can be used, inter alia, as a process technology for producing a product. The disclosed approach may be employed in the production of aerosols with bubble particles carrying a medicine which can be directed into human airways. Another possibility is the collection of generated aerosol bubbles on a plate or in a vessel to make a foam which can be further used for healthcare applications (e.g., medical diagnostics). Additionally, the disclosed approach may be employed for the drying/solidification of generated aerosol bubble particles and would make possible manufacturing in this way of solid spherical shells for pharmaceutical/material/cosmetics/food applications. Additionally, the disclosed approach may be employed for the drying/solidifying of a foam produced from the collected aerosol bubble particles to manufacture in this way solid foam materials.

Those skilled in the art will recognize, or be able to ascertain using no more than routine experimentation, many equivalents to the specific embodiments of the invention described herein. Such equivalents are intended to be encompassed by the following claims.

## Claims

1. A system for generating aerosols of particles having liquid shell of micron-scale thickness surrounding a gas core (aerosol bubbles), comprising:
a. a frame configured to create a falling film, the falling film having a thickness less than 20 µm, the falling film having a top portion, a bottom portion, and an intermediate portion;
b. a nozzle configured to direct a gas towards the intermediate portion of the falling film; and
c. a controller adapted to control the pressure of the gas provided to the nozzle, the pressure being between 10 mbar and 1500 mbar.

2. The system according to claim 1, further comprising a pump configured to provide a liquid to the top portion of the frame at a controlled rate.

3. The system according to claim 1, further comprising a camera directed at the intermediate portion.

4. The system according to claim 1, further comprising a container to collect a liquid from the bottom of the falling film.

5. The system according to claim 1, wherein a liquid used to create the falling film contains an additional material that is suspended or dissolved in the liquid.

6. The system according to claim 1, wherein the gas contains suspended micron- or nano-sized particles or droplets.

7. The system according to claim 1, wherein the nozzle has an inner diameter of less than 1 mm,
wherein a bank of nozzles blowing in parallel on a film is utilized,
wherein a carrier gas is supplied to dilute or direct the flow of the generated aerosols,
wherein the system is configured to generate monodisperse aerosols,
wherein the system is configured to generate bubbles in aerosols having an average diameter less than 1 mm,
wherein the system is configured to generate bubbles in aerosol having a maximum diameter less than 1 mm, and/or
wherein the system is configured to generate bubbles having a wall thickness of less than 5 µm.

8. The system according to claim 1, further comprising a second falling film.

9. The system according to claim 8, wherein the system is configured such that an aerosol bubble formed from the falling film is configured to pass through the second falling film, and/or
wherein the system is configured such that the falling film creates a first shell and the second falling film creates a second shell.

10. A method for generating aerosols of particles having liquid shell of micron-scale thickness surrounding a gas core (aerosol bubbles), comprising:
a. creating a falling film from a liquid containing a dissolved surfactant, the falling film having a thickness of less than 20 µm; and
b. generating a plurality of aerosol bubbles by providing a gas at a pressure of between 10 mbar and 1500 mbar to a nozzle configured to direct the gas through the falling film.

11. The method according to claim 10, further comprising drying the aerosol bubbles, solidifying the aerosol bubbles, or a combination thereof.

12. The method according to claim 10, wherein the provided gas contains micro or nanoparticles suspended in the gas, which are then encapsulated into the aerosol bubbles.

13. The method according to claim 10, further comprising collecting the plurality of aerosol bubbles.

14. The method according to claim 10, further comprising generating a liquid foam material from the plurality of aerosol bubbles, or generating a solid foam material from the plurality of aerosol bubbles.

15. The method according to claim 10, wherein the film is created by at least one of:
a. pumping liquid comprising a surfactant to the top of a vertically-oriented frame and allowing the liquid to create the film as it falls, or
b. periodically dipping frame into a liquid containing small amount of surfactant.

## Patentansprüche

1. System zur Erzeugung von Aerosolen aus Partikeln mit einer Flüssigkeitshülle im Mikrometerbereich, die einen Gaskern umgibt (Aerosolblasen), das Folgendes umfasst:
a. einen Rahmen, der so konfiguriert ist, dass er einen Fallfilm erzeugt, wobei der Fallfilm eine Dicke von weniger als 20 µm aufweist und einen oberen Abschnitt, einen unteren Abschnitt und einen mittleren Abschnitt aufweist;
b. eine Düse, die so konfiguriert ist, dass sie ein Gas auf den mittleren Abschnitt des Fallfilms richtet; und
c. eine Steuerung, die dazu ausgelegt ist, den Druck des der Düse zugeführten Gases zu steuern, wobei der Druck zwischen 10 mbar und 1500 mbar liegt.

2. System nach Anspruch 1, ferner umfassend eine Pumpe, die so konfiguriert ist, dass sie dem oberen Abschnitt des Rahmens eine Flüssigkeit mit einer kontrollierten Geschwindigkeit zuführt.

3. System nach Anspruch 1, ferner umfassend eine auf den mittleren Abschnitt gerichtete Kamera.

4. System nach Anspruch 1, ferner umfassend einen Behälter zum Auffangen einer Flüssigkeit vom Boden des Fallfilms.

5. System nach Anspruch 1, wobei eine zur Erzeugung des Fallfilms verwendete Flüssigkeit ein zusätzliches Material enthält, das in der Flüssigkeit suspendiert oder gelöst ist.

6. System nach Anspruch 1, wobei das Gas suspendierte Partikel oder Tröpfchen in Mikrometer- oder Nanogröße enthält.

7. System nach Anspruch 1, wobei die Düse einen Innendurchmesser von weniger als 1 mm aufweist,
wobei eine Reihe von Düsen verwendet wird, die parallel auf einen Film blasen,
wobei ein Trägergas zugeführt wird, um den Fluss der erzeugten Aerosole zu verdünnen oder zu lenken,
wobei das System so konfiguriert ist, dass es monodisperse Aerosole erzeugt,
wobei das System so konfiguriert ist, dass es Blasen in Aerosolen mit einem durchschnittlichen Durchmesser von weniger als 1 mm erzeugt,
wobei das System so konfiguriert ist, dass es Blasen im Aerosol mit einem maximalen Durchmesser von weniger als 1 mm erzeugt, und/oder
wobei das System so konfiguriert ist, dass es Blasen mit einer Wandstärke von weniger als 5 µm erzeugt.

8. System nach Anspruch 1, ferner umfassend einen zweiten Fallfilm.

9. System nach Anspruch 8, wobei das System so konfiguriert ist, dass eine aus dem Fallfilm gebildete Aerosolblase so konfiguriert ist, dass sie durch den zweiten Fallfilm hindurchgeht, und/oder
wobei das System so konfiguriert ist, dass der Fallfilm eine erste Hülle und der zweite Fallfilm eine zweite Hülle erzeugt.

10. Verfahren zur Erzeugung von Aerosolen aus Partikeln mit einer Flüssigkeitshülle im Mikrometerbereich, die einen Gaskern umgibt (Aerosolblasen), das Folgendes umfasst:
a. Erzeugen eines Fallfilms aus einer Flüssigkeit, die ein gelöstes Tensid enthält, wobei der Fallfilm eine Dicke von weniger als 20 µm aufweist; und
b. Erzeugen einer Vielzahl von Aerosolblasen durch Zuführen eines Gases mit einem Druck zwischen 10 mbar und 1500 mbar zu einer Düse, die so konfiguriert ist, dass sie das Gas durch den Fallfilm leitet.

11. Verfahren nach Anspruch 10, das ferner das Trocknen der Aerosolblasen, das Verfestigen der Aerosolblasen oder eine Kombination davon umfasst.

12. Verfahren nach Anspruch 10, wobei das bereitgestellte Gas im Gas suspendierte Mikro- oder Nanopartikel enthält, die dann in die Aerosolblasen eingekapselt werden.

13. Verfahren nach Anspruch 10, ferner umfassend das Sammeln der Vielzahl von Aerosolblasen.

14. Verfahren nach Anspruch 10, ferner umfassend das Erzeugen eines flüssigen Schaummaterials aus der Vielzahl von Aerosolblasen oder das Erzeugen eines festen Schaummaterials aus der Vielzahl von Aerosolblasen.

15. Verfahren nach Anspruch 10, wobei der Film durch mindestens eines der folgenden Verfahren erzeugt wird:
a. Pumpen einer Flüssigkeit, die ein Tensid umfasst, auf die Oberseite eines vertikal ausgerichteten Rahmens und Ermöglichen, dass die Flüssigkeit beim Herabfallen den Film bildet, oder
b. den Rahmen regelmäßig in eine Flüssigkeit tauchen, die eine geringe Menge an Tensid enthält.

## Revendications

1. Système de génération d'aérosols de particules ayant une enveloppe liquide d'épaisseur à l'échelle micrométrique entourant un noyau gazeux (bulles d'aérosol), comprenant :
a. un cadre configuré pour créer un film tombant, le film tombant ayant une épaisseur inférieure à 20 µm, le film tombant ayant une partie supérieure, une partie inférieure et une partie intermédiaire ;
b. une buse configurée pour diriger un gaz vers la partie intermédiaire du film tombant ; et
c. un dispositif de commande conçu pour commander la pression du gaz fourni à la buse, la pression étant comprise entre 10 mbar et 1 500 mbar.

2. Système selon la revendication 1, comprenant également une pompe configurée pour fournir un liquide à la partie supérieure du cadre à un débit commandé.

3. Système selon la revendication 1, comprenant également une caméra dirigée au niveau de la partie intermédiaire.

4. Système selon la revendication 1, comprenant également un récipient pour collecter un liquide à partir du fond du film tombant.

5. Système selon la revendication 1, dans lequel un liquide utilisé pour créer le film tombant contient un matériau supplémentaire qui est en suspension ou dissous dans le liquide.

6. Système selon la revendication 1, dans lequel le gaz contient des particules ou des gouttelettes en suspension de taille micrométrique ou nanométrique.

7. Système selon la revendication 1, dans lequel la buse a un diamètre interne inférieur à 1 mm,
dans lequel une banque de buses soufflant en parallèle sur un film est utilisée,
dans lequel un gaz porteur est fourni pour diluer ou diriger le flux des aérosols générés,
dans lequel le système est configuré pour générer des aérosols monodispersés,
dans lequel le système est configuré pour générer des bulles dans des aérosols ayant un diamètre moyen inférieur à 1 mm,
dans lequel le système est configuré pour générer des bulles dans un aérosol ayant un diamètre maximal inférieur à 1 mm, et/ou
dans lequel le système est configuré pour générer des bulles ayant une épaisseur de paroi inférieure à 5 µm.

8. Système selon la revendication 1, comprenant également un second film tombant.

9. Système selon la revendication 8, dans lequel le système est configuré de sorte qu'une bulle d'aérosol formée à partir du film tombant est configurée pour traverser le second film tombant, et/ou
dans lequel le système est configuré de sorte que le film tombant crée une première enveloppe et le second film tombant crée une seconde enveloppe.

10. Procédé de génération d'aérosols de particules ayant une enveloppe liquide d'épaisseur à l'échelle micrométrique entourant un noyau gazeux (bulles d'aérosol), comprenant :
a. la création d'un film tombant à partir d'un liquide contenant un tensioactif dissous, le film tombant ayant une épaisseur inférieure à 20 µm ; et
b. la génération d'une pluralité de bulles d'aérosol en fournissant un gaz à une pression comprise entre 10 mbar et 1 500 mbar à une buse configurée pour diriger le gaz à travers le film tombant.

11. Procédé selon la revendication 10, comprenant également le séchage des bulles d'aérosol, la solidification des bulles d'aérosol, ou une combinaison de ceux-ci.

12. Procédé selon la revendication 10, dans lequel le gaz fourni contient des micro ou nanoparticules en suspension dans le gaz, qui sont ensuite encapsulées dans les bulles d'aérosol.

13. Procédé selon la revendication 10, comprenant également la collecte de la pluralité de bulles d'aérosol.

14. Procédé selon la revendication 10, comprenant également la génération d'un matériau en mousse liquide à partir de la pluralité de bulles d'aérosol, ou la génération d'un matériau en mousse solide à partir de la pluralité de bulles d'aérosol.

15. Procédé selon la revendication 10, dans lequel le film est créé par au moins l'un des éléments suivants :
a. le pompage d'un liquide comprenant un tensioactif vers le haut d'un cadre orienté verticalement et le fait de permettre au liquide de créer le film pendant qu'il tombe, ou
b. l'immersion périodique du cadre dans un liquide contenant une petite quantité de tensioactif.
